Europäisches Patentamt

European Patent Office

Office européen des brevets

(11) Publication number: **0 180 670**
**A1**

(12) # EUROPEAN PATENT APPLICATION

(21) Application number: **84307526.8**

(22) Date of filing: **01.11.84**

(51) Int. Cl.⁴: **C 12 M 1/00**
**C 07 C 7/11, B 01 D 53/14**

(43) Date of publication of application:
**14.05.86 Bulletin 86/20**

(84) Designated Contracting States:
**AT BE CH DE FR GB IT LI LU NL SE**

(71) Applicant: **CRYOTEC ENERGY SYSTEMS CO., LTD.**
**Recorder Works Grindle Road**
**Coventry West Midlands CV6 6BX(GB)**

(72) Inventor: **Jury, Kenneth James**
**6 Brinklow Road**
**Binley Coventry CV3 2HY(GB)**

(72) Inventor: **Eden, Robert David**
**4, Crackley Cottages**
**Kenilworth Coventry CV8 2FG(GB)**

(74) Representative: **Allard, Susan Joyce et al,**
**BOULT, WADE & TENNANT 27 Furnival street**
**London EC4A 1PQ(GB)**

(54) Recovery of biogas.

(57) Biogas, essentially consisting of 50-70% methane and 30-50% $CO_2$, is scrubbed to remove the $CO_2$ by two spray scrubbers (8, 14) arranged in series along the gas flow path. The spray scrubbers (8,14) atomise an absorbent liquid which is regenerated and returned to the scrubbers (8, 14). The absorbent liquid is preferably water which is fed under pressure to a regeneration unit (23) where the water is atomised and its pressure is dropped to ambient pressure so forcing out the $CO_2$ which is vented (30) to the atmosphere. The water is cooled in a chiller (33) to enhance the solubility of $CO_2$ therein and returned to the scrubbers (8,14). The water returning to the first scrubber (8) is thoroughly mixed with incoming biogas in a mixing chamber (6) before entering the scrubber (8). The raw biogas is preferably pre-washed (3) to remove solids and compressed (4) before entering the scrubbers and polished (39) after being scrubbed to remove any remaining contaminants. The polished gas may be cooled (48), liquefied (49) and stored in cryogenic tanks (50).

./...

FIG. 2.

— WATER
— METHANE

## RECOVERY OF BIOGAS

The present invention relates to the recovery of gas, particularly biogas, and to an apparatus and method for the recovery of biogas and, optionally, its subsequent liquefaction.

Methane gas is produced in large quantities in, for example, landfill sites, and certain sewage works. It is currently recovered in a few locations but the bulk of this potentially valuable energy reserve is lost.

Methane gas produced from these sites may be scrubbed and liquefied to obtain liquefied methane gas (LMG) which can be substituted for fuel oil in burners and diesel engines as an efficient and low-polluting fuel.

It has been estimated that landfill sites alone in Britain could provide up to 4000 million $m^3$ of methane gas per year. This represents an energy equivalent of 130 PJ/year. There is also a large potential for the recovery and liquefaction of biogas from anaerobic sewage digesters as well as animal
wastes and crop residues.

The recovered gas is a superb fuel for converted diesel engines and there are commercially available engines able to run on 15% diesel and 85% vaporised LMG. Such engines have higher thermal efficiency and power output than conventional diesel engines, as well as reduced noise level and reduced emission of pollutants. Alternatively the gas may be burnt directly in burners (in Western Europe 15% of the total energy used is derived from natural gas) and, in the longer term, it is envisaged that methane may be used as a chemical feedstock.

It is thus apparent that the economic recovery of gas from the sources mentioned above would be highly significant, both economically and environmentally.

Biogas (methane) is produced by the anaerobic digestion of organic waste together with large quantities of carbon dioxide (about 30%-50% of the gas volume may be $CO_2$) and smaller quantities of sulphides, and even vinyl chloride from landfill sites, as well as other impurities. The gas must be removed with mechanical work and then scrubbed before it can be used. The purified biogas can be piped to a nearby energy demand, used for on-site power generation, compressed and transported in cylinders or liquefied in cryogenic tanks.

The last option appears to have the widest potential.

Hitherto, exploitation of biogas has been limited particularly because no equipment is available for economically processing the gas into a transportable form. Desirably the equipment itself should be transportable since, in many cases (e.g. landfill sites), the gas source will only produce viable quantities of gas for a limited time and the installation of permanent plant would not be justified.

The liquefaction of gas is a well established technology used, for example, in the liquefaction of natural gas. However, the provision of a viable process for cleaning biogas remains a problem. Natural gas typically contains only about 1.1 mole% $CO_2$ and is processed by permanent plant so the technology used for cleaning natural gas is not directly applicable to biogas.

Carbon dioxide is commonly removed from natural gas by contacting the gas with a suitable absorbent liquid, such as an aqueous solution of an alkanolamine or potassium carbonate. The absorbent solution may be regenerated by steam stripping and reused.

A regenerative procedure for cleaning biogas has been proposed in which the biogas is cleaned in a packed tower (the gas is passed up a packed column

- 4 -

0180670

against a stream of water at a pressure of 10 bar) and the $CO_2$ - containing water is regenerated in two stages: firstly its pressure is dropped to 1 bar and secondly it is air stripped in a packed tower ["Studie uber die Eignung von Biogas als Treibstoff für Handwirtschaftstraktoren" (Reports of the Swiss Federal Research Station for Farm Management and Agricultural Engineering), J. Frankhauser and A. Moser, 1983, pp 167-169].

However, the proposal in the above Swiss study does not offer an economic way of recovering biogas. The Swiss study proposes to use packed towers as in covential natural gas scrubbing but, for anything other than the smallest scale operations, this would require the assembly of large fixed plant, which is not only expensive and environmentally deleterious but is also totally unsuited to the limited life of landfill sites, which would render the fixed plant superfluous in a relatively short time.

In particular, such apparatus requires a large water flow rate and for practical purposes a large fixed water reservoir is necessary, and even then a relatively high level of $CO_2$ remains in the biogas. For example, it is believed that such apparatus would use a water flow rate of 50 gals (227 litres)/min for a biogas inflow rate of 100 cfm ($2.8m^2$/min) and have a residual $CO_2$ level of at least 2%.

Thus, it would be desirable to provide a relatively compact apparatus for the regenerative scrubbing of biogas, that is, an apparatus in which the relatively large amount of $CO_2$ in biogas could be removed by relatively small scrubbers and in which the $CO_2$ - absorbent is regenerated to obviate the need for continuous disposal and re-supply of absorbent. Such apparatus would be most useful if it were transportable.

In particular, it would be desirable to provide such

0180670

apparatus in combination with liquefaction apparatus. It would further be desirable to provide apparatus for scrubbing biogas which is self-contained and requires no external services so that it can be used in remote locations.

We have now found that a relatively small apparatus for scrubbing biogas can be provided by two-stage spray-scrubbing of the biogas with an atomised carbon dioxide-absorbent liquid and regeneration of the absorbent liquid and, thus, in one aspect the present invention resides in an apparatus for recovering biogas, comprising in series along the gas flow path first stage and second stage spray scrubbers for scrubbing biogas with an atomised liquid absorbent for $CO_2$, and regeneration means arranged to regenerate the absorbent liquid which has passed through the first scrubber and the absorbent liquid which has passed through the second scrubber and for the regenerated absorbent liquid to be returned to the first and second scrubbers.

In a second aspect, there is provided a method of recovering biogas, comprising passing the gas sequentially through first and second stage spray scrubbers in which a $CO_2$ - absorbent liquid is atomised to remove $CO_2$ from the biogas, regenerating the absorbent liquid which has passed through the first scrubber and the absorbent liquid which has passed through the second scrubber and returning the regenerated liquid to the scrubbers for atomisation.

The $CO_2$-absorbent liquid may be any          liquid conventionally used for gas "sweetening" and the liquid will be regenerated in an appropriate way. However, the use of, for example, an alkanolamine or $K_2CO_3$ solution is somewhat disadvantageous since, not only must the expense of obtaining the alkanolamine or $K_2CO_3$ be undertaken, but also steam stripping is required for regeneration which is undesirable because of the need for a local water supply and the consumption of energy during steam - generation. Moreover, absorbent solutions are corrosive.

It is a further advantage of our apparatus and method that they may be operated using water as the absorbent and this constitutes a preferred embodiment of the process.

Although the process which uses water as the absorbent is not restricted to any particular process of regeneration, it is advantageously achieved by dropping the pressure of pressurised $CO_2$-contaminated water and atomising it. Typically, the water will have been pressurised by a pump before it is passed through the scrubbers. This method of regeneration enables the provision of compact regeneration means comprising a spray unit in which the water pressure is dropped to ambient pressure and the water is atomised. The $CO_2$ lost from the water is vented to the atmosphere. Thus, in one preferred embodiment, pump means is provided for pressurising the absorbent liquid (which in use will be water) to be passed through the scrubbers and the regeneration means comprises means to drop the pressure of the absorbent liquid and atomise it.

It is not essential for all the water to be atomized and we have found that up to 10% (preferably not more than 5%) of the contaminated water may have its pressure dropped in the regeneration means without atomisation.

This preferred method of stripping is energy-efficient and does not require the provision of a water supply. It is envisaged that air stripping could otherwise be used or the $CO_2$ could be driven off by heating the $CO_2$-contaminated water.

Turning now to the two stage scrubbing process, we have found that a substantial proportion of the $CO_2$ can be removed by this process leaving no more than a few percent and desirably about 0.8% of the biogas as $CO_2$. The remaining $CO_2$ can be completely removed for practical purposes by a gas polisher.

The bulk of the $CO_2$ may be removed in the first stage leaving the second stage to remove a substantial portion of the remainder. Using water as the absorbent, the second scrubber should ideally atomise the water to droplets having a size no larger than 20 $\mu$m to achieve a satisfactory level of $CO_2$ absorption. Indeed, it is by the achievement of such a fine atomisation in the second scrubber that a sufficient level of $CO_2$ absorption may be attained for a really compact scrubber system to be used. A minumum droplet size of substantially 5 μm is preferred because little is gained by carrying out finer atomisation (which is difficult to obtain) and we most prefer atomisation to be in the range of 10 μm in the second scrubber as an optimum size: a larger droplet size is more inefficient but a smaller one is excessively troublesome to achieve. Of course, the desired atomisation sizes have to be considered in practical terms and not too strictly because a range of droplet sizes is inevitably produced.

The first scrubber need not atomise the water so finely as the second one since it is removing the $CO_2$ down to a higher concentration than that which it is desirable to achieve in the second scrubber. Typically, the maximum droplet size should not exceed 500 μm to achieve a sufficient level of absorption and little is gained by using a droplet size of less than 100 μm. We have found a droplet size in the region of 300 μm to be most satisfactory.

When water is used as the absorbent, it is most preferable to use one or more hydraulic nozzles in the first stage scrubber and one or more sonic nozzles in the second stage scrubber, in order to achieve the desired atomisation sizes. However, sonic nozzles may be used in both scrubbers.

The operation of the hydraulic nozzle(s), if used, may be enhanced by thoroughly mixing the biogas and the absorbent liquid prior to passing them through the hydraulic nozzle(s). Thus, in a preferred embodiment of the invention the first scrubber comprises a hydraulic nozzle and is preceded by a gas/liquid mixer; more preferably the second scrubber comprises a sonic nozzle.

Indeed, the use of a gas/liquid premixer before a hydraulic nozzle is believed to be novel and we further provide an invention which is an apparatus for recovering biogas comprising a gas/liquid mixer having an inlet for biogas and an inlet for absorbent liquid and a scrubber comprising a hydraulic nozzle downstream of the gas/liquid mixer. Such apparatus is desirably incorporated into the apparatus of the aforesaid invention comprising two scrubber stages but this is not essential to achieve sufficient cleaning of the gas, although a more efficient and compact apparatus may be obtained if the two scrubber apparatus is used.

It is also preferred for a pre-wash (desirably regenerative) to be provided upstream of the scrubbers and the mixing chamber (if any) to wash solids out of the gas stream. Preferably the wash is a hot water wash and the water is desirably centrifuged to remove the solids and thus regenerate it.

The apparatus of the invention preferably comprises means to liquefy the scrubbed gas and in one embodiment there is provided a fully integrated scrubber/liquefier wherein there is a multi-stage (i.e. 2 or more stage) compressor connected upstream of an expansion unit through which, in use, the biogas is expanded to liquefy it and the first and second scrubbers of the invention are each located between successive stages of the compressor. This embodiment is particularly compact and it is envisaged that where compactness is at a premium, then self-containment regeneration of the absorbent liquid may not be necessary. Accordingly, there is further provided the invention which resides in an apparatus for the recovery of biogas comprising a multi-stage compressor arranged upstream of an expansion unit and two spray scrubbers arranged in series along the gas flow path and each located between two successive stages of the compressor. The scrubbers may be adjacent each other or separated by a stage of the compressor.

On the other hand, a more self-contained apparatus may be obtained if it is adapted to be powered by scrubbed biogas after an initial start up period. Conveniently, the apparatus may be powered by biogas in combination with diesel as a fuel for modified diesel engines capable of running on diesel/methane mixtures.

An important characteristic of biogas is that it

- 11 -    0180670

may contain a relatively large proportion of $CO_2$, typically between 30 and 50%. One problem addressed by the present invention is the provision of apparatus capable of removing this relatively high proportion of $CO_2$ which is both relatively compact and with capacity to scrub an economically viable rate of flow of gas while at the same time enabling the absorbent to be re-used . It is envisaged that the process of the invention could, therefore, be advantageously applied to the removal of one or more components of a gas mixture by a liquid absorbent selective thereto, which components constitute from 20% to 70% of the volume of the gas mixture. Accordingly, there is further provided a method of washing from a gas mixture one or more components thereof which form from 20% to 70% by volume of the gas mixture, which process comprises passing the gas mixture sequentially through first and second stage spray scrubbers in which an absorbent liquid selective to the components to be washed out is atomised, regenerating the absorbent liquid which has passed through the first scrubber and the absorbent liquid which has passed through the second scrubber and returning the regenerated liquid to the scrubbers for atomisation.

The present invention is further described by way of example only with reference to the accompanying

- 12 -

0180670

drawings, in which:

Figure 1 is a flow diagram illustrating a process for liquefying biogas using apparatus according to the invention;

Figure 2 is a diagrammatic outline of the apparatus used in the process illustrated in Figure 1; and

Figure 3 is a detailed diagram of the scrubber circuit of the apparatus of Figure 2.

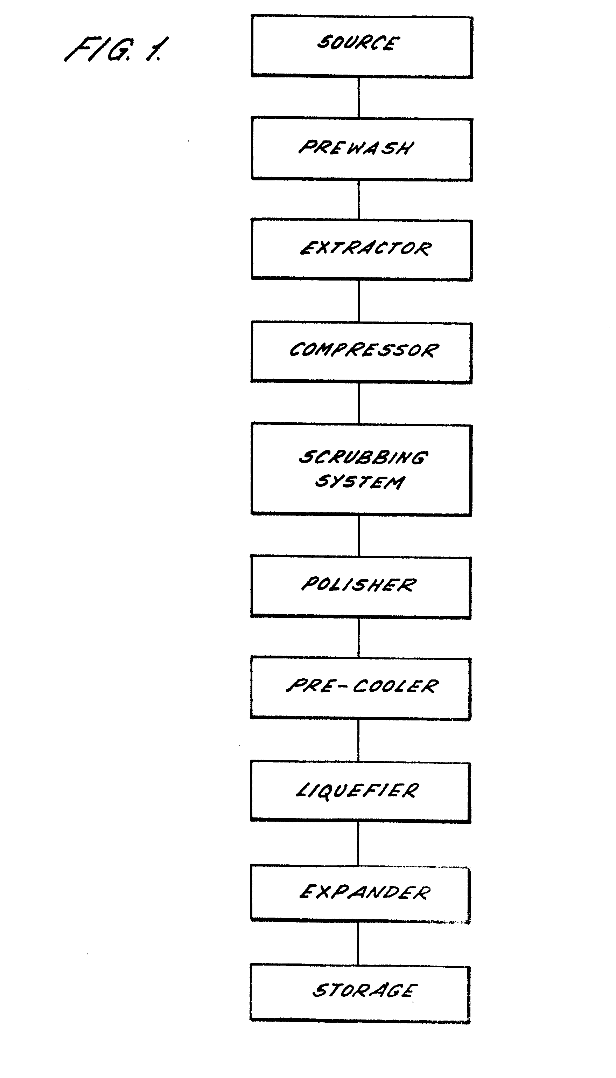

Referring now to Figure 1, biogas is produced by the bacterial degradation of waste in a landfill site or of another mass of organic waste. Typically, 30-50% of the gas produced is $CO_2$ and a small amount (no more than a few percent) is other impurity gases, especially $H_2S$, and the remainder is methane. Solid particles are also contained in the gas.

The biogas is extracted from the source, for example a landfill site, through piping extending thereinto. Any extraction arrangement may be used but generally there is a series of wells as described below with lateral piping leading to a centralised extractor or vacuum pump. The wells consist of perforated pipes extending vertically into the waste to a point roughly three-quarters of the total depth of the landfill site. The boreholes in which the pipes are situated are backfilled with rock or gravel to prevent waste from blocking the perforations and the perforations are in the lower portion of the wall to prevent induction of air into the landfill site. A concrete seal is typically placed above the gravel. Plastics pipes are generally used because the gas is corrosive.

The biogas is drawn from the source to the extractor optionally via a prewash to remove solids

and a little $CO_2$. As an alternative to using a prewash, there may be used an extractor which removes detritus, From the extractor the gas passes to a compressor which pressurises the gas before it enters the scrubbing system which removes the bulk (e.g. 98%) of the $CO_2$. The residual impurities are removed in a polisher. The illustrated system is one in which the scrubbed gas is liquefied, stored in cryogenic tanks and after being polished the gas is passed to a pre-cooler prior to liquefaction. The liquefier is optionally followed by an expander to reduce the pressure of the liquefied gas which is then stored in cryogenic tanks.

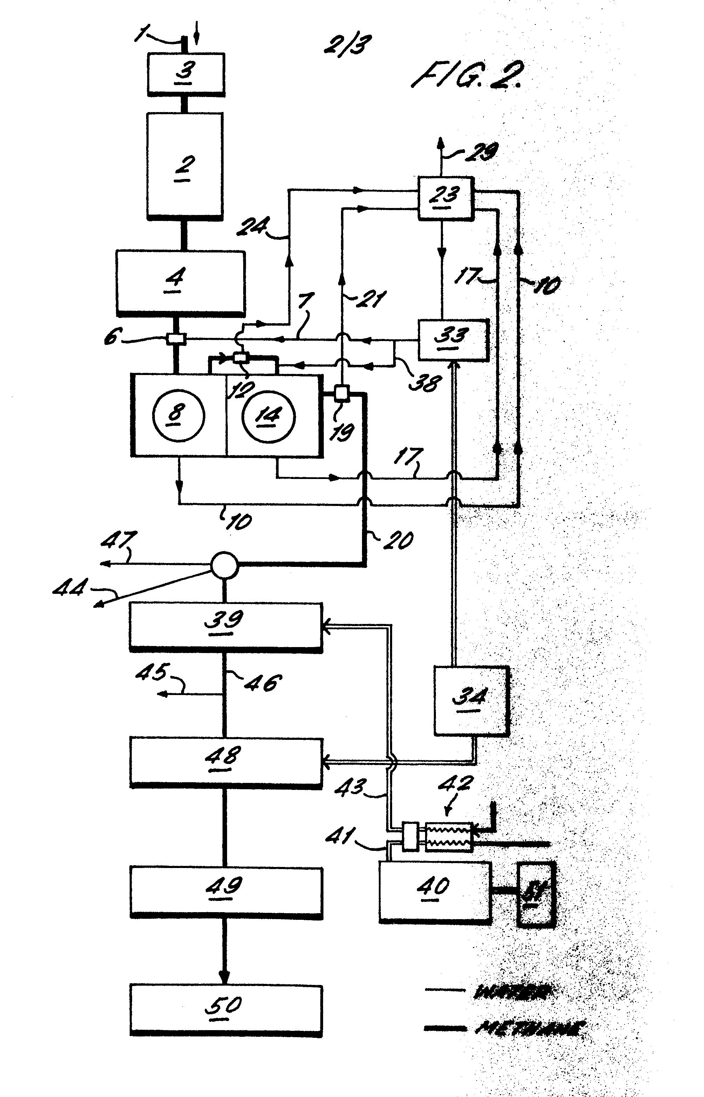

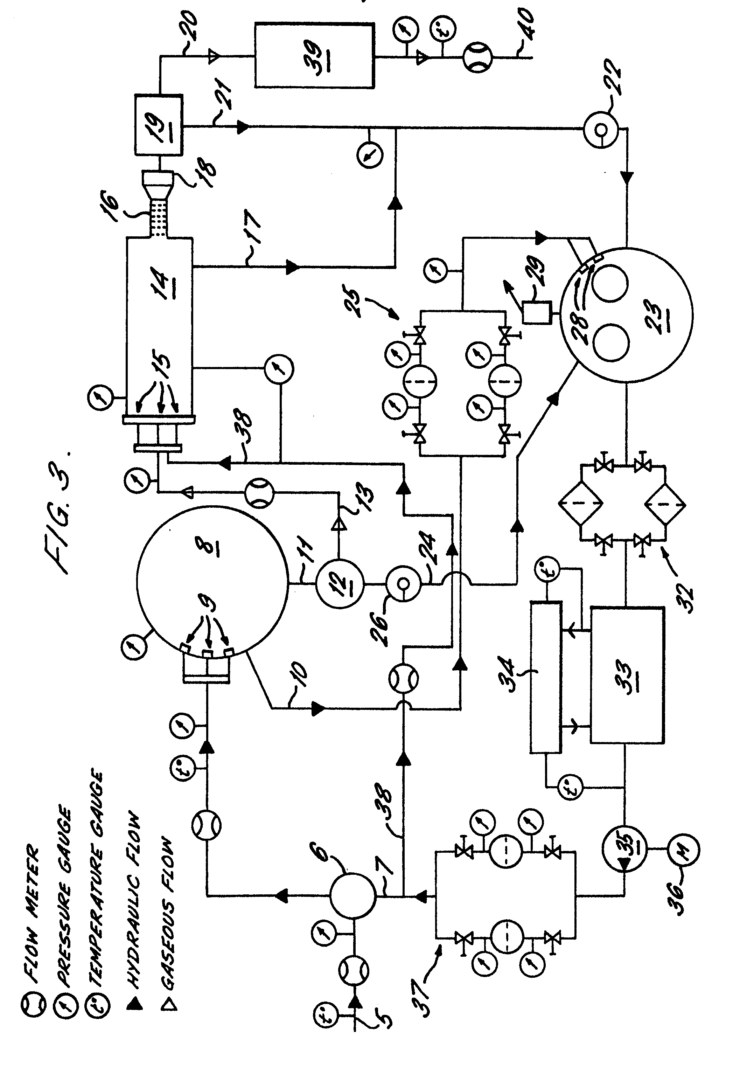

Turning now to Figures 2 and 3, the illustrated apparatus will be described in detail.

Contaminated biogas is drawn through an inlet duct 1 by an extractor turbine 2. Before passing into the extractor the gas passes through a prewash device 3 to remove solids, e.g. organic detritus. The prewash is preferably a hot water wash effected by passing the gas up a tower counter-current to a spray of hot water. The dirty water at the bottom of the tower 3 is cleaned by centrifugation (a technique known per se) and passed to an immersion heater to be heated before being re-sprayed. It is extremely desirable to use this regenerative cleaning process because it avoids the need for a local water supply, although a small amount of water will be lost and have to be replaced from time to time when the bottom of the tower 3 is cleaned to remove sludge. The hot wash tower 3 need be no more than 2 metres high.

The type of extractor used is not critical but it may be a centrifugal fan or gas blower. The prewash may be dispensed with if an extractor is used which itself removes solids from the gas stream and to this end a liquid ring compressor may be used. However, such compressors are relatively inefficient and we prefer not to use them.

From the extractor 2, the gas passes to a compressor 4 to compress the gas prior to scrubbing. Preferably the gas is pressured to 20-40 bar, most preferably in the region of 30-35 bar. Any suitable compressor may be used.

The compressor pumps the gas through a pipe 5 to a mixing chamber 6, which forms part of the scrubber circuit shown in detail in Figure 3. Also pumped to the mixing chamber is chilled (e.g. $2^{O}C$), clean water through an inlet duct 7, the gas being at a slightly greater pressure than the water.

The gas and the water are thoroughly mixed in the mixing chamber.

In the mixing chamber of the illustrated

embodiment the gas is introduced into the water stream through a sieve which breaks the gas up into small bubbles.

The gas/water mixture flows to a first stage spray scrubber 8 which removes the bulk of the $CO_2$, typically about 80% thereof. The first stage scrubber 8 may conveniently be provided with hydraulic nozzles 9 to obtain suitable atomisation of the water. The size of the atomised droplets is not critical but if they have a size (diameter) in excess of about 500 μm the level of $CO_2$ absorbance is impaired and little advantage is gained by atomisation to less than 100 μm. We have found the optimum atomisation size to be in the region of 300 μm.

Hydraulic nozzles are known in the art and detailed description thereof is not necessary.

The first stage scrubber 8 has an outlet 10 for bulk water which collects in the bottom of the scrubber and an outlet 11 for the mist of biogas and atomised water which is now laden with $CO_2$. The outlet 11 leads to a gas/liquid separator 12 from which the biogas is exhausted through a duct 13 leading to the second stage spray scrubber 14. Clean pressurised water enters the second scrubber 14 from a duct 38.

The second scrubber 14 should, for most applications, reduce the amount of $CO_2$ to a small

0180670

fraction of its original concentration, for example to about 2% of its original level (typically equivalent to 0.8 to 1% of the total gas volume) and the $CO_2$ can be removed most efficiently by atomising the water in the second scrubber 14 to a fine size. The optimum droplet size of the atomised water is in the region of 10 $\mu$m and a preferred size range is of from 5 to 20 $\mu$m. It is not worthwhile to reduce the droplet size to below about 5 $\mu$m whilst efficiency is noticeably impaired when the droplet size exceeds 20 $\mu$m.

We prefer that the second scrubber be provided with sonic nozzles 15, since these can atomise the water to the desired size and are commercially available. Suitable nozzles may be purchased from Ultrasonics Limited.

The gas/water mist passes from the body of the second scrubber 14 up a column 16 of perforated plates arranged above the second scrubber 14 which serve to separate out some of the water particles and further mix the gas and water. The gas/water mist then passes to a mist eliminator 18 which is arranged above the column 16 of perforated plates and filled with inert particles to catch the water droplets. Bulk water from the column 16 and the mist eliminator 18 falls back to the bottom of the second scrubber 14 from which it flows out through a bulk water outlet duct 17. After passing through the mist eliminator 18 the gas still contains entrained water droplets and passes to a second gas/water separator 19 to finally remove the water droplets.

The scrubbed gas is exhausted from the second gas/ water separator 19 through a duct 20 whilst the water leaves the second separator 19 through water outlet duct 21 to which the bulk water outlet duct 17 of the second scrubber is connected and passes via a float trap 22 to a water regeneration unit 23. Also fed to the regeneration unit 23 is water collected by the bulk water outlet of the first scrubber 8, the water separated out by the first gas/water separator 12 which passes to the regeneration unit 23 via a duct 24 and the bulk water which leaves the second scrubber 14 by outlet duct 17. Thus, all the water used in the scrubbers is fed to the water regeneration unit 23.

Interposed in the duct 10 between the first scrubber 8 and the regeneration unit 23 is a high pressure filter 25 to filter out any solid particles washed out of the biogas in the first scrubber 8 whilst a float trap 26 is provided in the duct leading to the regeneration unit 23 from the first gas/water separator 12. The float traps 22 and 26 collect the water passing through the connected ducts until the water level reaches a desired level at which a valve is automatically triggered to pass the water to the regeneration unit 23.

The gas/water separators 12 and 19 are preferably centrifugal separators comprising a metal (e.g. nickel or nickel chrome alloy) foam disc rotating at several thousand r.p.m. The water droplets are caught by the foam disc and thrown out to circumferential edge of the disc where the water is collected.

The $CO_2$-contaminated water which is fed to the water regeneration unit 23 enters the unit 23 through hydraulic spray heads 28 which atomise the water. The regeneration unit 23 has a vent to the atmosphere and the water entering the regeneration unit 23 is still under pressure (typically at least 20 bar) and the combination of atomisation and pressure drop forces the $CO_2$ out of the water. Air is blown onto the mist in the regeneration unit 23 to drive it into a third gas/water separator 29 (which may be a centrifugal separator as described above) from which the air and the $CO_2$ are vented to the atmosphere through the vent 30. In the illustrated embodiment the third gas/water separator 29 is located in the same housing as the regeneration unit 23 and the vent 30 of the separator 29 also acts as the vent of the regeneration unit 23.

In a modification of the illustrated apparatus the ducts 21 and 24 are not provided with spray heads 28 because only a small fraction (e.g., up to 10% of the $CO_2$-contaminated water passes through them and satisfactory regeneration can be achieved with a spray head provided only for the duct 10. In this case the pressure of the water entering from the ducts 21 and 24 will still be dropped.

From the gas/water separator 29 the water is passed through a low pressure filter 32 to a chiller 33 to cool the water and thus enhance its capacity to absorb $CO_2$. Ideally, the water is chilled to about 2°C but the temperature in practice will typically range up to 4° or 5°C.

The chiller unit 33 is a heat exchanger in which the water is cooled by refrigerant from a refrigeration plant 34. The cooled water passes to a pump 35 driven by a motor 36 which raises the water pressure for it to be passed through the scrubbers 8 and 14. The water pressure, as indicated above, will be slightly less than the pressure of the gas entering the mixing chamber 6.

From the pump 35 the water flowsthrough a filter system 37 of in-line strainers to remove any particles which may still be entrained and one portion enters the mixing chamber 6 via the duct 7 whilst another portion flows to the second scrubber 14 along the duct 38.

The scrubbed biogas which has been exhausted from the scrubbing circuit through the duct 20 passes to a polisher 39 for the removal of any remaining contaminant gases (e.g. $H_2S$, $CO_2$ and oxygen) which have not been washed out by the scrubbers. The polisher 39 may contain a molecular sieve and activated charcoal. The polished gas leaves the polisher 39 through an outlet duct 40 for its desired ultimate use.

A portion of the cleaned gas is generally used to power the recovery apparatus since this reduces the need for a separate fuel supply. This portion of gas may leave the system through a pipe 45 connected to the outlet duct 46 of the polisher 39. In Figure 2 there is shown an engine 40 capable of running on a diesel/methane mixture which drives a generator 41 which in turn generates the electricity to power the apparatus . Of course, more than one engine 40 may be used and one or more engines may be mechanically coupled to units of the apparatus to drive them directly. Also, arrangements must be made to power the apparatus during an initial start up period before biogas is available and it is convenient to use engines capable of being switched from diesel to a diesel/methane mixture for this purpose. The switching may be effected manually or automatically upon detection of methane by a suitably placed detector.

The polisher 39 must be regenerated once it is saturated with contaminants and this may be effected by passing hot gas through the polisher 39. The hot gas is conveniently hot air heated by recovered biogas and, for the sake of efficiency, the hot air is heated, or at least pre-heated, by the hot exhaust  fumes from the engine(s) 40. Figure 2 illustrates an arrangement in which exhaust fumes travel along a duct 41 from the engine 40 and through a turbo-heat exchanger 42 counter-current to a stream of clean air in an air intake duct 43.

If desired, the air may be further heated before it enters the polisher 39 from which it is vented to the atmosphere, for example through vent 44 shown in Figure 2. Clearly the polisher 39 must be sealed off

from the rest of the system to carry out regeneration and in the embodiment of Figure 2 this would require closing down the entire apparatus. However, if two polishers are arranged in parallel the biogas may be passed through one while the other is regenerated to permit continuous operation. It is possible to determine when a polisher is saturated by the use of detectors to ascertain when one or more contaminant gases is passing through the polisher but we have found it easiest to effect cyclinq between the two-polishers by a preset timing mechanism.

In addition to the polisher 39, one or two pre-polishers (not shown) selective to $H_2S$ may be inserted into the gas flow path between the hot wash tower 3 and the extractor 2. The advantage of using a pre-polisher is that it removes the corrosive and toxic $H_2S$ from the biogas before the biogas has passed through the bulk of the apparatus. Such a prepolisher may contain granular iron oxide or activated charcoal as the absorbent.

Especially if such a pre-polisher is used, the biogas for powering the recovery apparatus may be taken from the gas stream before it enters the polisher 39, and a pipe for this purpose is indicated by numeral 47 of Figure 2. The pipe 47 may also be used to conduct away gas to be piped to any other local point of consumption, indeed if all the gas is to be piped away for local use the polisher 39 may be dispensed with entirely, especially if an $H_2S$ pre-polisher is installed. As to whether the biogas must be polished, and as to the extent to which it must be polished, will depend on the local conditions and requirements, including the level of contaminants in the raw biogas.

If the gas is going to be stored in compressed gas cylinders or as a liquid in cryogenic tanks a high standard of purity will be required and it is envisaged that the polisher 39 would always be used.

Any suitable apparatus may be used to compress or liquefy the biogas and known conventional apparatus would be appropriate. Figure 2 illustrates in outline apparatus for liquefying the biogas. From the polisher 39 it passes to a pre-cooler 46 which may be connected to the refrigeration plant 34 coupled to the chiller 33 of the scrubber circuit, as shown in Figure 2. The cooled gas passes to a liquefier 49 and optionally through an expander (see Figure 1) to reduce its pressure before being stored in cryogenic tanks 50.

It will be seen that the illustrated embodiment provides a substantially self-contained apparatus for recovering biogas. The only external supplies needed are a small amount of water to replace losses from the water regeneration unit 23 and the prewash unit 3 and a quantity of diesel to power the apparatus during the initial start up period and to be used as a co-fuel with methane during subsequent running. By powering the apparatus with an available motor capable of running on a 90% methane/10% diesel mix only very occasional supplies of diesel will be needed to fill the diesel tank. Likewise a water reservoir can be provided to minimise the need for water to be supplied during the running of the apparatus.

The use of two spray scrubbers to clean the biogas enables the apparatus to be compact and we have found that a recovery and liquefaction apparatus as illustrated in the drawings and capable of a biogas inflow rate of 100 cfm (2.8 $m^2$/min ) and of delivering as much as 400 gallons (1818 litres)/day of methane at 1 bar and 112 K may be housed in two standard 20 foot long (6.1m) freight containers. The freight containers can readily be transported from site to site so that the apparatus can be economically installed even at remote sites. This apparatus is designed for the gas leaving the second scrubber typically to have a residual $CO_2$ level of about 0.8% with a water flow rate of 15-20 gallons (68-90l)/min.

The apparatus may be adapted to be remotely controlled via a MODEM unit or other suitable telephone link and a microprocessor unit. This will mean that no personnel will be required to monitor its operation and it can be remotely interrogated by telephone (suitably via a radio link) to ascertain its performance.

Many modifications of the illustrated apparatus will be apparent to the skilled person. For example, in one preferred embodiment there is provided a fully integrated scrubber/liquefier in which the biogas is compressed through a four stage compressor [for example to a pressure of 3,500psi (240 bar)]. The scrubbing would be carried out by the two stage, regenerative, spray scrubbing apparatus of the invention, between the second and third stages of the compressor and polished. The fully compressed gas would then be expanded through a refrigeration unit and thus liquefied.

What is essential to the invention is that it has two spray scrubbers in series with regeneration of the $CO_2$-absorbent used in each scrubber for the regenerated absorbent to be recycled through each scrubber. This enables a compact apparatus which does not require a continuous supply of fresh absorbent. Preferably the absorbent is regenerated by a self-contained process which does not require the input of any material (except insofar as fuel may be required to drive the apparatus) and also it is preferred for any pre-wash system to be regenerative. Ideally the $CO_2$-absorbent is water and for the optimum level of $CO_2$ absorption the second spray scrubber atomises the water to a size of from 5 to 20 μm. The operation of the apparatus can be further enhanced by the provision of a mixing chamber upstream of the first scrubber.

CLAIMS:

1. An apparatus for recovering biogas, comprising in series along the gas flow path first stage and second stage spray scrubbers for scrubbing biogas with an atomised liquid absorbent for $CO_2$, and regeneration means arranged to regenerate the absorbent liquid which has passed through the first scrubber and the absorbent liquid which has passed through the second scrubber and for the regenerated absorbent liquid to be returned to the first and second scrubbers.

2. An apparatus as claimed in Claim 1 wherein the first scrubber is capable of atomising the absorbent liquid to droplets having a size of from 100 to 500 μm and the second scrubber is capable of atomising the absorbent liquid to droplets having a size of from 5 to 20 μm.

3. An apparatus as claimed in Claim 2 wherein the first scrubber is capable of atomising the absorbent liquid to droplets having a size in the order of 300 μm and the second scrubber is capable of atomising the absorbent liquid to droplets having a size in the order of 10 μm.

4. An apparatus as claimed in any one of the

- 27 -

0180670

preceding claims wherein the first scrubber comprises a hydraulic nozzle to atomise the absorbent liquid and the second scrubber comprises a sonic nozzle to atomise the absorbent liquid.

5. An apparatus as claimed in any one of the preceding claims which is arranged for scrubbed biogas to power the apparatus after an initial start up period.

6. An apparatus as claimed in any one of the preceding claims wherein there is provided pump means for pressurising the absorbent liquid to be passed through the scrubbers and the regeneration means comprises means to drop the pressure of the absorbent liquid and atomise it.

7. An apparatus as claimed in any one of claims 1 to 5 wherein the regeneration means comprises means to drop the pressure of the absorbent liquid and atomise it, which apparatus comprises:

an extractor for extracting biogas from the source and pressurising it,

pump means connected to the regeneration means for pressurising regenerated absorbent liquid to a pressure less than that of the pressurised biogas,

a mixing chamber connected to the extractor and the pump means downstream thereof for mixing the biogas and the absorbent liquid and to the first stage scrubber upstream thereof,

a first gas/liquid separator connected in the gas flow path between the first and second scrubbers and having a liquid outlet connected to the regeneration means, for separating the gas and the absorbent liquid after they have passed through the first scrubber,

a duct connecting the pump means and the second scrubber for regenerated water to be pumped to the second scrubber,

a second gas/liquid separator downstream of the second scrubber having a liquid outlet connected to the regeneration means and a gas outlet,

a gas polishing means connected to the gas outlet of the second gas/liquid separator, and

a chiller connected between the regeneration means and the pump means for cooling the absorbent liquid passing from the regeneration means to the pump means.

8. A method of recovering biogas, comprising passing the gas sequentially through first and second stage spray scrubbers in which a $CO_2$- absorbent liquid is atomised to remove $CO_2$ from the biogas, regenerating the absorbent liquid which has passed through the first scrubber and the absorbent liquid which has passed through the second scrubber and returning the regenerated liquid to the scrubbers for atomisation.

9 . A method as claimed in Claim 9 wherein in the first scrubber the absorbent liquid is atomised by a hydraulic nozzle and in the second scrubber it is atomised by a sonic nozzle and the biogas and the absorbent liquid are mixed prior to entering the first scrubber.

10. A method as claimed in Claim 9 or Claim 10 wherein the recovered biogas is liquefied.

11. An apparatus for recovering biogas comprising a gas/liquid mixer having an inlet for biogas and an inlet for absorbent liquid and a scrubber comprising a hydraulic nozzel downstream of the gas/liquid mixer.

12. An apparatus for recovering biogas comprising a multi-stage compressor arranged upstream of an expansion unit and two spray scurbbers arranged in series along the gas flow path and each located between two successive stages of the compressor.

13.  A method of washing from a gas mixture one or more components thereof which form of from 20% to 70% by volume of the gas-mixture, which process comprises passing the gas mixture sequentially through first and second stage spray scrubbers in which an absorbent liquid selective to the components  to be washed out is atomised, regenerating the absorbent liquid which has passed through the first scrubber and the absorbent liquid which has passed through the second scrubber and returning the regenerated liquid to the scrubbers for atomisation.

0180670

*FIG. 1.*

```
        ┌─────────────┐
        │   SOURCE    │
        └──────┬──────┘
               │
        ┌──────┴──────┐
        │   PREWASH   │
        └──────┬──────┘
               │
        ┌──────┴──────┐
        │  EXTRACTOR  │
        └──────┬──────┘
               │
        ┌──────┴──────┐
        │ COMPRESSOR  │
        └──────┬──────┘
               │
        ┌──────┴──────┐
        │  SCRUBBING  │
        │   SYSTEM    │
        └──────┬──────┘
               │
        ┌──────┴──────┐
        │  POLISHER   │
        └──────┬──────┘
               │
        ┌──────┴──────┐
        │ PRE-COOLER  │
        └──────┬──────┘
               │
        ┌──────┴──────┐
        │  LIQUEFIER  │
        └──────┬──────┘
               │
        ┌──────┴──────┐
        │  EXPANDER   │
        └──────┬──────┘
               │
        ┌──────┴──────┐
        │   STORAGE   │
        └─────────────┘
```

*F/G. 2.*

FIG. 3.

FLOW METER
PRESSURE GAUGE
TEMPERATURE GAUGE
HYDRAULIC FLOW
GASEOUS FLOW

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GENIE CHIMIQUE, vol. 90, no. 3, September 1963, pages 74-76; P. ROUSSEY: "Epuration, sous pression, des gaz à teneurs élevées en anhydride carbonique et acide sulfhydrique" * Pages 74-76 * | 1,6,8, 13 | C 12 M 1/00 C 07 C 7/11 B 01 D 53/14 |
| X | FR-A-1 205 615 (CHEMICAL CONSTRUCTION CORP.) * Page 4, right-hand column, abstract; figure * | 1,6,8, 13 | |
| A | | 4 | |
| A | DE-A-3 024 352 (K. GEIGER) * Page 3, claim 8; figure * | 7 | |
| | | | **TECHNICAL FIELDS SEARCHED (Int. Cl.4)** |
| A | US-A-4 157 958 (B.H. CHOW) | | |
| A | EP-A-0 102 131 (SEAC INTERNATIONAL) * Page 4, lines 12-20 * | 4 | C 07 C B 01 D C 10 K C 12 M |

The present search report has been drawn up for all claims

| Place of search | Date of completion of the search | Examiner |
|---|---|---|
| THE HAGUE | 04-07-1985 | PYFFEROEN K. |